# FASCICULE DE BREVET EUROPEEN

(11) **EP 1 483 978 B1**
(45) Date de publication et mention de la délivrance du brevet: **19.04.2006**
(21) Numéro de dépôt: 04291382.2
(22) Date de dépôt: 03.06.2004
(51) Int. Cl.: A23L 1/304

(54) **Procédé biologique d'obtention d'une preparation alimentaire à base de fer hémique ainsi que préparation alimentaire obtenue suite à la mise en oeuvre de ce procédé**
Biologisches Verfahren zur Herstellung eines Hämeisenpräparats und daraus hergestellte Nahrungsmittelprodukte
Biological process for the production of a food product comprising heme-iron and food product obtained with said process

(30) Priorité: 04.06.2003 FR 0306720
(43) Date de publication de la demande: 08.12.2004
(73) Titulaire: Compagnie Generale de Dietetique, 14050 Caen (FR)
(72) Inventeur: Nedjaoum, Fouzia, 51000 Reims (FR); Dhulster, Pascal, 59650 Villeneuve d'ascq (FR); Fatah, Nouria, 59175 Vendeville (FR); Guillochon, Didier, 59700 Marcq en Baroeuil (FR); Legrand, Charles, 14000 Caen (FR); Lepley-Legrand Marie-Pascale, 14000 Caen (FR)
(74) Mandataire: Cabinet HERRBURGER

(56) Documents cités:
- LEBRUN F ET AL: "Influence of molecular interactions on ultrafiltration of a bovine hemoglobin hydrolysate with an organic membrane" JOURNAL OF MEMBRANE SCIENCE, ELSEVIER SCIENTIFIC PUBL.COMPANY. AMSTERDAM, NL, vol. 146, no. 1, 22 juillet 1998 (1998-07-22), pages 113-124, XP004128606 ISSN: 0376-7388
- IN MAN-JIN ET AL: "Process development for heme-enriched peptide by enzymatic hydrolysis of hemoglobin" BIORESOURCE TECHNOLOGY, vol. 84, no. 1, août 2002 (2002-08), pages 63-68, XP0001157182 & ISSN: 0960-8524
- LEBRUN F ET AL: "Solubility of heme in heme-iron enriched bovine hemoglobin hydrolysates." JOURNAL OF AGRICULTURAL AND FOOD CHEMISTRY 46 (12) 5017-5025 1998 CORRESPONDENCE (REPRINT) ADDRESS, D. GUILLOCHON, LAB. DE TECH. DES SUBSTANCES NATURELLES, IUT A LILLE I, BP 179, 59653 VILLENEUVE D'ASCQ, CEDEX, FRANCE. FAX (33) 3 20 43 44 72. E-MAI, 1998, XP0001157184
- UZEL CONNIE ET AL: "Absorption of heme iron" SEMINARS IN HEMATOLOGY, vol. 35, no. 1, janvier 1998 (1998-01), pages 27-34, XP0009024511 & ISSN: 0037-1963
- DATABASE WPI Section Ch, Week 198438 Derwent Publications Ltd., London, GB; Class B04, AN 1984-234698 XP002267905 & JP 59 140834 A (MITSUBISHI CHEM IND LTD) 13 août 1984 (1984-08-13)
- DATABASE WPI Section Ch, Week 198950 Derwent Publications Ltd., London, GB; Class D12, AN 1989-366812 XP002267906 & JP 01 273560 A (ICHIMARU PHARCOS INC) 1 novembre 1989 (1989-11-01)
- DATABASE WPI Section Ch, Week 200041 Derwent Publications Ltd., London, GB; Class B04, AN 2000-466468 XP002267907 & CN 1 252 997 A (TIANJIN IND MICROBES INST) 17 mai 2000 (2000-05-17)

## Description

La présente invention concerne une préparation alimentaire à teneur élevée en complexes fer hémique - peptides solubles en milieu acide destinée à la prévention et au traitement de la carence en fer ainsi qu'un procédé biologique permettant l'obtention de cette préparation.

La carence en fer est un problème de santé majeur commun aux pays industrialisés et aux pays en voie de développement qui touche environ 20 % de la population mondiale.

Le fer qui est l'oligo-élément quantitativement le plus important dans l'organisme est en effet indispensable au maintien de la vie cellulaire. Il est en particulier essentiel pour le transport de l'oxygène et des électrons et la synthèse de l'ADN et constitue un élément actif de nombreuses molécules qui jouent un rôle vital dans une large gamme de fonctions cellulaires parmi lesquelles on peut citer l'hémoglobine (transport de l'oxygène), les cytochromes (transporteurs d'électrons dans les chaînes respiratoires), et les enzymes hémiques (catalase, peroxydase).

Les apports nutritionnels conseillés en fer (mg par jour) sont les suivants :

| | |
|---|---|
| 6-12 mois | 7 |
| 1-3 ans | 7 |
| 4-9 ans | 7 |
| 10-12 ans | 8 |
| Adolescents 13-19 ans | 12 |
| Adolescentes 13-19 ans | 14 |
| Hommes adultes | 9 |
| Femmes réglées | 16 |
| Femmes ménopausées | 9 |
| Femmes allaitantes | 10 |
| Femmes enceintes | 25-35 |

Le fer provenant de l'alimentation existe sous deux formes qui sont absorbées par les entérocytes du jéjunum par un mécanisme d'absorption différent : le fer non hémique et le fer hémique.

Le fer hémique est présent dans deux protéines alimentaires, l'hémoglobine et la myoglobine que l'on trouve majoritairement dans la viande rouge.

L'hème est constitué par un atome de fer situé au centre d'une structure cyclique organique non protéique plane renfermant quatre noyaux pyrroliques réunis par des ponts méthényles ainsi que huit chaînes latérales.

L'absorption du fer hémique (de 20 à 30 %) est largement supérieure à celle du fer non hémique car elle est peu influencée par les facteurs alimentaires et les sécrétions gastriques.

Le fer non hémique constitue environ 90 % du fer présent dans l'alimentation : en effet 60 % du fer d'origine animale et la totalité du fer d'origine végétale sont non hémiques ; on le trouve dans les céréales, les légumes secs, les fruits, les produits laitiers et les légumes.

L'absorption du fer non hémique est plus faible que celle du fer hémique (de 2 à 20 %) et varie avec la composition du régime alimentaire. Elle peut être stimulée par certains facteurs alimentaires ou à l'inverse inhibée par d'autres.

Différents traitements de la carence en fer sont actuellement proposés par les spécialistes (modification du régime alimentaire, fortification en fer de la nourriture ou encore supplémentation en fer).

Il existe actuellement sur le marché toute une gamme de préparations à base de sels de fer (sulfate de fer, gluconate de fer, fumarate de fer...) destinées au traitement et à la prévention de la carence en fer.

Cette source de fer non hémique présente toutefois toute une gamme d'inconvénients parmi lesquels on peut noter :
- une variation de son absorption avec la composition du régime alimentaire
- une mauvaise tolérance digestive (caractère astringent des sels de fer ayant pour conséquence l'induction de troubles intestinaux dans 20 % des cas, ce qui fait que le traitement ne peut pas être poursuivi.)
- une action oxydante (risque de génération in situ dans le côlon de radicaux libres dont les effets néfastes ne sont plus à démontrer).

Le but de l'invention consiste à proposer une préparation alimentaire adaptée au traitement et à la prévention de la carence en fer de nature à remédier à ces inconvénients et présentant une excellente bio-disponibilité.

Il a pu être établi que le fer hémique constitue la forme de fer la plus facilement assimilable et entraîne moins d'effets indésirables que le fer minéral.

L'hémoglobine constitue une source de fer hémique intéressante, vu qu'il s'agit là d'un sous-produit facile à obtenir et de faible prix de revient.

Il n'est toutefois pas envisageable pour des raisons diététiques d'utiliser l'hémoglobine en supplémentation du fait qu'elle ne renferme en proportion pondérale que 0,34 % de fer, la fraction restante étant constituée par des protéines.

Par suite, l'administration d'hémoglobine à doses thérapeutiques serait susceptible d'engendrer un déséquilibre de la balance protéique, en particulier dans les pays industrialisés où les régimes alimentaires sont déjà très, voire trop riches en protéines : 6 g d'hémoglobine pure devraient en effet être ingérés pour délivrer la dose journalière de fer recommandée.

Compte tenu de cette situation, il serait souhaitable de pouvoir disposer pour le traitement des carences en fer de préparations à base de fer hémique issu de l'hémoglobine ayant une teneur en fer largement supérieure à 0,34 % (p/p).

De nombreux procédés ont déjà été proposés pour tenter d'obtenir de telles préparations.

Aucun de ces procédés ne s'est toutefois jusqu'à présent avéré pleinement satisfaisant en raison de problèmes de bio-disponibilité.

Afin d'augmenter la teneur en fer de l'hémoglobine, certaines équipes ont dissocié l'hème de la globine par des procédés utilisant des solvants organiques qui ont tous abouti à des préparations fortement concentrées en fer hémique. L'absorption du fer hémique de ces préparations est cependant limitée.

Selon la publication Uzel C. et Conrad M.E. (1998). Absorption of heme iron. Semin. Hematol. 35, 27-34, des études réalisées chez l'homme ont permis d'établir que l'hème est très mal absorbé s'il est administré séparé de la globine. Il a été établi que l'hème non transporté par la globine forme des agrégats qui précipitent au pH acide de l'estomac et ne sont pas absorbés par l'intestin. Il semblerait que les produits de dégradation de l'hémoglobine, c'est-à-dire les peptides jouent un rôle dans l'absorption du fer hémique en le complexant, limitant ainsi son agrégation.

Selon la publication Schümann K., Elsenhans B. et Mäurer A. (1998). Iron supplementation. J. Trace Elements Med. Biol. 12, 129-140, l'addition de protéines entières lors de l'administration de l'hème semble augmenter sa bio-disponibilité.

Ces différents travaux ont donc permis d'établir que la bio-disponibilité du fer hémique semble être liée à son état de complexation : le fer hémique isolé forme des agrégats qui sont insolubles au pH acide de l'estomac et n'est donc pas absorbé par l'intestin alors que le fer hémique complexé à des protéines (comme dans le cas de l'hémoglobine) ou à des peptides forme peu d'agrégats et est totalement soluble au pH acide de l'estomac ; il est donc absorbé.

Compte tenu de cette situation, les spécialistes ont cherché à proposer des préparations à base d'hème complexé à des peptides ou à des protéines de façon à permettre sa solubilisation au pH acide de l'estomac.

Plusieurs équipes ont ainsi développé des procédés combinant l'hydrolyse enzymatique de l'hémoglobine et l'ultrafiltration des hydrolysats peptidiques issus de cette hydrolyse dans le but de préparer des fractions de peptides hémiques ayant une-concentration en fer plus élevée que celle de l'hémoglobine.

Jusqu'à présent on n'a toutefois pas proposé de procédé qui permette de préparer des fractions de peptides hémiques avec un rapport fer hémique / peptides important et dont l'hème soit complexé à des peptides de grande taille qui limitent son état d'agrégation et permettent de le solubiliser à pH acide.

A titre d'exemple, selon la publication Eriksson C. (1982). Heme-iron enriched amino acid and a process for the preparation of heme-iron-enriched amino acid. et le document US-4 411 915, on a déjà proposé un procédé de préparation, à partir du sang d'animaux, par exemple de sang de porc ou de sang de boeuf ou encore de ses dérivés tels que le cruor, d'une préparation composée d'hème et de courtes séquences peptidiques dont le rapport fer/peptides est jusqu'à quinze fois plus élevé que dans l'hémoglobine.

Cette préparation enrichie en fer provient d'une hydrolyse poussée de l'hémoglobine dénaturée. L'enrichissement est obtenu par centrifugation ou par ultrafiltration ou en combinant la centrifugation et l'ultrafiltration.

Toutefois, l'hème présent dans cette fraction est sous forme très fortement agrégée, ce qui limiterait son absorption. En effet l'hème est complexé à des peptides de très petite taille qui ne sont pas capables d'empêcher la précipitation de ces gros agrégats d'hème à pH acide.

Bien qu'il ait été ainsi possible d'obtenir un enrichissement en fer important par rapport à l'hémoglobine (jusqu'à 15 fois dans certaines conditions), ce fer n'est pas potentiellement biodisponible car complexé à des peptides de petite taille incapables d'empêcher sa précipitation à pH acide.

A titre d'autre exemple, selon le document KR 10-2000-0053778 et la publication In M.J., Chae H.J. et Oh N.S. (2002). Process development for heme-enriched peptide by enzymatic hydrolysis of hemoglobin. Biores. Technol. 84, 63-68, on a également décrit un procédé de préparation, à partir de l'hémoglobine, d'une fraction peptidique à teneur élevée en fer.

Ce procédé de préparation est basé sur une hydrolyse très poussée de l'hémoglobine par un mélange de protéases et sur une ultrafiltration.

On a obtenu par ce procédé un enrichissement qui peut atteindre 14 fois.

Malheureusement, le fer hémique de cette préparation est lié à des peptides de petite taille incapables de l'empêcher de former des agrégats et donc de le maintenir en solution en milieu acide.

Selon la publication Lebrun F., Bazus A., Dhulster P. et Guillochon D. (1998). Influence of molecular interactions on ultrafiltration of a bovine hemoglobin hydrolysate with an organic membrane. J. Membr. Sci. 146, 113-124, on a déjà décrit un procédé permettant la préparation, à partir de sang d'animaux, par exemple de sang de porc ou de sang de boeuf ou encore de ses dérivés tels que le cruor d'un hydrolysat de peptides hémiques contenant 2,5 fois plus de fer que l'hémoglobine et dans lequel l'hème est lié à des peptides de grande taille.

La préparation élaborée par ce procédé contient des complexes hème - peptides solubles dans une large gamme de pH y compris à pH acide ; ces peptides de grande taille possèdent la même capacité que la globine de l'hémoglobine à limiter l'agrégation de l'hème et à le transporter en milieu acide.

L'enrichissement en fer ainsi obtenu est toutefois notablement insuffisant.

Enfin, selon la publication Seligman P.A., Moore G.M. et Rhoda B.S. (2000). Clinical studies of HIP: an oral heme-iron product. Nutr. Res. 20, 1279-1286, on a déjà testé chez l'homme une préparation à base de fer hémique contenant une proportion de fer de 1 %, soit trois fois plus que l'hémoglobine native, mais dont seulement la moitié est soluble à pH acide. Il a été constaté que cette préparation présente chez l'homme une bio-disponibilité largement supérieure à celle de l'hème concentré.

Malheureusement, tous les procédés décrits précédemment aboutissent soit à des préparations riches en fer hémique, mais dont le fer hémique est insoluble à pH acide, soit à des préparations avec un fer hémique soluble, mais dont la teneur en fer n'est pas assez élevée.

Dans ce contexte, l'invention s'est donné pour but de proposer une préparation alimentaire à base de fer hémique potentiellement biodisponible adaptée au traitement de la carence en fer, de nature à combler cette lacune.

La présente invention a donc pour objet un procédé de préparation d'une fraction de peptides hémiques à teneur élevée en fer hémique dont la majorité du fer hémique est complexé à des peptides de grande taille.

Compte tenu du fait que cette préparation est destinée à être utilisée en tant que complément alimentaire, il est essentiel que ce procédé soit un bio-procédé n'utilisant aucun solvant.

Selon l'invention, ce procédé est caractérisé par la succession des étapes suivantes :
- on soumet une première fraction de sang d'animaux, par exemple de sang de porc ou de sang de boeuf ou encore de ses dérivés tels que le cruor à une hydrolyse enzymatique ménagée de façon à obtenir un premier hydrolysat renfermant du fer hémique complexé à des peptides de grande taille ainsi que des populations peptidiques de petite taille non complexées au fer hémique,
- on enrichit le premier hydrolysat ainsi obtenu en complexes fer hémique - peptides par ultrafiltration de façon à éliminer des populations peptidiques non complexées au fer hémique, et à obtenir une solution d'hydrolysat enrichie en complexes fer hémique - peptides dans laquelle le fer hémique est complexé à des peptides de grande taille qui permettent sa solubilisation à pH acide,
- on soumet parallèlement une seconde fraction de sang d'animaux, par exemple de sang de porc ou de sang de boeuf ou encore de ses dérivés tels que le cruor à une hydrolyse enzymatique poussée de façon à obtenir un second hydrolysat renfermant du fer hémique complexé à des petits peptides, ainsi que des populations peptidiques de petite taille non complexées au fer hémique,
- on centrifuge le second hydrolysat ainsi obtenu afin d'éliminer une proportion importante (environ 60 %) des petits peptides non liés à l'hème, et d'obtenir un culot de centrifugation très concentré en hème,
- on solubilise le culot de centrifugation notamment par addition de soude, de façon à obtenir une solution extrinsèque d'hème dans laquelle l'hème est lié à des peptides de petite taille et est par suite totalement insoluble à pH acide,
- on mélange la solution d'hydrolysat enrichi en complexes fer hémique - peptides et la solution extrinsèque d'hème de façon à permettre la fixation de l'hème extrinsèque sur les peptides de grande taille de la solution d'hydrolysat enrichi et la libération des petits peptides liés à l'hème extrinsèque,
- on centrifuge le mélange ainsi obtenu afin d'éliminer l'hème extrinsèque qui n'a pas été fixé aux peptides de grande taille, et
- on enrichit la solution ainsi obtenue en complexes fer hémique - peptides par ultrafiltration de façon à éliminer des petits peptides libérés lors de l'étape de mélange.

Le procédé conforme à l'invention est par suite basé sur une propriété particulière de certains complexes fer hémique - peptides qui sont aptes à fixer des quantités supplémentaires d'hème.

Ce procédé consiste donc à effectuer parallèlement l'hydrolyse enzymatique de deux fractions de sang d'animaux, par exemple de sang de porc ou de sang de boeuf ou encore de ses dérivés tels que le cruor, à savoir d'une part une hydrolyse ménagée d'une première fraction permettant l'obtention d'une fraction peptidique dans laquelle le fer hémique est complexé à des peptides de grande taille qui permettent la solubilisation ultérieure à pH acide, et d'autre part une hydrolyse poussée d'une seconde fraction permettant l'obtention d'une fraction peptidique renfermant des peptides de petite taille, dont une partie est complexée à l'hème.

L'hydrolyse poussée est suivie d'une étape d'élimination d'environ 60 % des petits peptides non complexés à l'hème par centrifugation de façon à obtenir une fraction peptidique fortement concentrée en fer hémique (solution d'hème extrinsèque). Ce fer hémique est ensuite fixé sur les peptides de grande taille obtenues à l'issue de l'hydrolyse ménagée.

Il semblerait que la fixation de l'hème extrinsèque sur les peptides de grande taille s'effectue par interactions hydrophobes.

Il est à noter que l'hème extrinsèque est totalement insoluble à pH acide mais est ultérieurement rendu soluble grâce à sa fixation sur les gros peptides.

Ce procédé permet, après purification, d'obtenir une préparation alimentaire très fortement enrichie en fer par rapport à l'hémoglobine, soluble entre pH 1 et pH 3 et entre pH 5,5 et pH 12, et dans laquelle 80 % du fer est complexé à des peptides et donc potentiellement bio-disponible.

Selon l'invention, la fraction de peptides hémiques à teneur élevée en fer finale peut être sous forme liquide mais est avantageusement soumise à une étape de séchage de façon à obtenir une préparation alimentaire sous forme de poudre adaptée au traitement de la carence en fer.

Une telle poudre peut avantageusement être incorporée dans des gélules destinées à être absorbées en tant que complément alimentaire.

Le procédé de séchage utilisé conformément à l'invention (four sous vide et broyage, atomisation ou lyophilisation et broyage), conditionne les propriétés (écoulement, dispersibilité, ...) des poudres finalement obtenues.

Le procédé de séchage au four sous vide suivi d'un broyage s'est avéré le plus avantageux dans le cas où la poudre doit ensuite être introduite dans des gélules.

Selon l'invention, l'hydrolyse enzymatique ménagée et l'hydrolyse enzymatique poussée des deux fractions de sang d'animaux, par exemple de sang de porc ou de sang de boeuf ou encore de ses dérivés tels que le cruor de départ sont de préférence effectuées avec une enzyme active à pH acide, notamment de la pepsine porcine.

Il est à noter que la pepsine porcine présente de nombreux avantages.

Il s'agit en effet là d'une enzyme digestive permettant de réaliser des hydrolyses à pH acide, pH qui permet d'éviter les contaminations bactériennes. De plus, la réaction enzymatique peut aisément être arrêtée au stade souhaité en remontant le pH jusqu'à 8 (pH de dénaturation de la pepsine) notamment par addition de soude diluée.

Selon l'invention, on a pu établir que la réaction d'hydrolyse enzymatique ménagée et la réaction d'hydrolyse enzymatique poussée devaient de préférence être mises en oeuvre à un pH inférieur à 6.

Le pH du sang d'animaux, par exemple du sang de porc ou de sang de boeuf ou encore de ses dérivés tels que le cruor peut être amené à la valeur désirée par addition d'acide chlorhydrique.

A titre d'exemple si la matière première de départ est du cruor, lors de l'étape d'hydrolyse enzymatique ménagée, on ajuste de préférence le degré d'hydrolyse a une valeur inférieure ou égale à 5 %, alors que lors de l'étape d'hydrolyse enzymatique poussée, on ajuste le degré d'hydrolyse à 15 % et plus.

Le degré d'hydrolyse correspond au pourcentage de liaisons peptidiques qui sont coupées ; les spécialistes savent parfaitement maîtriser ce paramètre en particulier par dosage des molécules NH₂ libérées.

Le choix des degrés d'hydrolyse correspond à une caractéristique déterminante du procédé conforme à l'invention.

En effet, on a pu établir qu'en deçà d'une certaine taille, les peptides ne peuvent plus permettre la solubilisation de l'hème à pH acide et en outre la fixation de quantités supplémentaires d'hème extrinsèque.

Par suite, lors de l'hydrolyse ménagée, il est impératif de veiller à obtenir un enrichissement en fer suffisant de la première fraction de sang d'animaux, par exemple de sang de porc ou de sang de boeuf ou encore de ses dérivés tels que le cruor, tout en ne diminuant pas trop fortement là taille des peptides qui complexent l'hème.

Lors de l'hydrolyse poussée, il est impératif d'obtenir un hème suffisamment pur mais néanmoins lié à des peptides de petite taille qui permettent sa fixation sur les peptides de grande taille de la fraction obtenue à l'issue de l'hydrolyse ménagée, en empêchant toute agrégation trop importante de nature à nuire à cette fixation.

Selon une caractéristique préférentielle de l'invention, lors de l'étape de mélange de la solution d'hydrolysat enrichi en complexes fer hémique - peptides et de la solution extrinsèque d'hème, on met en oeuvre des quantités de solutions telles que la quantité d'hème apportée par la solution d'hème extrinsèque soit jusqu'à dix fois plus importante que la quantité d'hème de la solution d'hydrolysat.

Selon l'invention, le mélange des deux solutions est en règle générale effectué sous agitation et à température ambiante.

L'étape d'enrichissement par ultrafiltration permet ensuite d'éliminer de la préparation les petits peptides libérés lors de l'étape de mélange.

Selon une autre caractéristique de l'invention, lors des étapes d'enrichissement par ultrafiltration, on utilise des membranes hydrophiles ayant un seuil de coupure d'environ 10 kDa.

On a pu établir que l'utilisation de telles membranes permet l'élimination des peptides non complexés à l'hème avec un taux de rétention de l'hème proche de 100 %.

Selon l'invention, il est particulièrement avantageux de mettre en oeuvre au moins une, de préférence trois étapes de dia filtration sur des membranes identiques après chacune des étapes d'enrichissement par ultrafiltration, ce de façon à augmenter l'enrichissement de l'hydrolysat en poursuivant l'élimination des peptides.

On a pu vérifier qu'au-delà de trois étapes de dia filtration on n'observe plus d'enrichissement notable.

Conformément à l'invention, il est par ailleurs avantageux de centrifuger le premier hydrolysat avant son enrichissement par ultrafiltration de façon à éliminer les peptides insolubles qui se rassemblent dans le culot de centrifugation.

On a pu vérifier que cette centrifugation n'entraîne pas de perte d'hème et permet d'augmenter notablement le temps de colmatage des membranes d'ultrafiltration.

L'invention permet pour la première fois de proposer un procédé permettant d'obtenir à partir du sang d'animaux par exemple du sang de porc ou du sang de boeuf ou encore de ses dérivés tels que le cruor une préparation alimentaire à base de fer hémique renfermant à la fois :
- une teneur importante en fer pouvant être jusqu'à dix fois supérieure à celle d'une préparation d'hémoglobine, et
- un fer hémique se trouvant sous une forme facilement assimilable.

De plus, l'invention permet également pour la première fois de proposer un procédé permettant d'enrichir des hydrolysats enzymatiques de sang d'animaux par exemple de sang de porc ou de sang de boeuf ou encore de ses dérivés tels que le cruor en fer hémique assimilable d'un facteur pouvant aller jusqu'à dix.

L'invention concerne également une préparation alimentaire à base de fer hémique adaptée au traitement de la carence en fer et notamment obtenue suite à la mise en oeuvre du procédé susmentionné.

Une telle préparation alimentaire peut se présenter sous une forme liquide ou sous la forme d'une poudre de peptides hémiques ayant un rapport pondéral fer / peptides supérieur à 2 %, de préférence d'environ 3 %, plus de 80 % de ce fer étant complexé aux peptides.

Une telle poudre est soluble au pH acide de l'estomac.

L'invention permet donc de proposer un complément alimentaire pulvérulent présentant une bonne bio disponibilité, et de surcroît très fortement enrichi en fer par rapport à l'hémoglobine.

Selon une caractéristique particulièrement avantageuse de l'invention, la masse volumique tassée de cette poudre est proche de 1.

L'invention se rapporte également à une préparation alimentaire se présentant sous la forme de gélules ayant par exemple une contenance volumique d'environ 0,5 ml qui sont remplies de poudre de peptides hémiques.

De telles gélules peuvent avantageusement être gastro-protégées.

Ce mode de formulation permet de protéger la préparation de peptides hémiques contre des hydrolyses pepsiques non contrôlées au niveau de l'estomac.

Ces hydrolyses aboutiraient en effet à la formation de peptides de petite taille et d'acides aminés incapables de solubiliser les agrégats d'hème au pH acide de l'estomac.

L'invention permet donc d'apporter aux consommateurs la dose journalière de fer recommandée en leur faisant absorber quotidiennement trois gélules ayant une contenance d'environ 0,5 ml, ce qui correspond à un mode de formulation acceptable.

La mise en oeuvre du procédé conforme à l'invention suivie d'un séchage de la fraction de peptides hémiques à teneur élevée en fer obtenue par le four sous vide a à titre d'exemple permis d'obtenir une poudre ayant les caractéristiques physico-chimiques rassemblées dans le tableau ci-dessous.

| | |
|---|---|
| % Fer (p / p) | 2,1 |
| % Fer complexé (p / p) | 1,7 |
| % Peptides (p / p) | 75 |
| Rapport Fer / Peptides % (p / p) | 2,8 |
| Masse volumique tassée | 0,80 |

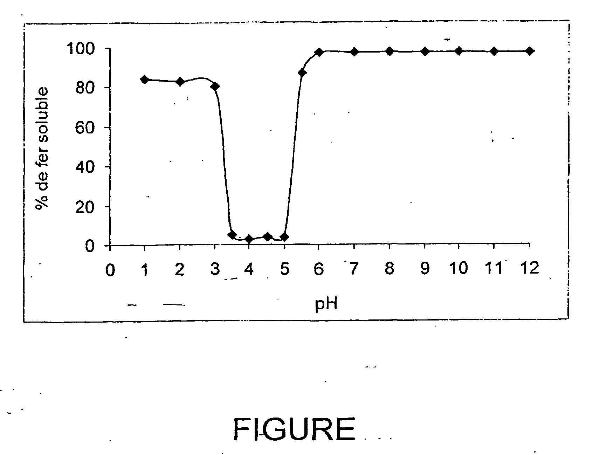

La courbe de solubilité du fer de cette fraction de peptides hémiques séchés en fonction du pH est représentée sur la figure jointe en annexe.

Pour obtenir cette courbe, des solutions à 0,5 % (p / v) ont été préparées par dilution de la poudre dans des solutions tampons de pH allant de 1 à 12. Ces solutions ont été centrifugées et la quantité de fer présente dans le surnageant a été dosée. Cette courbe montre que le fer de cette fraction peptidique est bien soluble dans la zone de pH acide (pH 1 à pH 3), confirmant ainsi un transport de l'hème par les peptides. On note une zone de pH comprise entre 3,5 et 5 où le fer n'est pas soluble. Cette zone de précipitation correspond au pHi des peptides. Ces derniers entraînent l'hème qu'ils transportent lors de leur précipitation

## Revendications

1. Procédé biologique d'obtention d'une préparation alimentaire à base de fer hémique adaptée au traitement de la carence en fer à partir de sang d'animaux, par exemple de sang de porc ou de sang de boeuf ou encore de ses dérivés tels que le cruor,
**caractérisé par** la succession des étapes suivantes :
- on soumet une première fraction de sang d'animaux, à une hydrolyse enzymatique ménagée de façon à obtenir un premier hydrolysat renfermant du fer hémique complexé à des peptides de grande taille ainsi que des populations peptidiques non complexées au fer hémique,
- on enrichit le premier hydrolysat ainsi obtenu en complexes fer hémique - peptides par ultrafiltration de façon à éliminer des populations peptidiques non complexées au fer hémique, et à obtenir une solution d'hydrolysat enrichie en complexes fer hémique - peptides,
- on soumet parallèlement une seconde fraction de sang d'animaux, à une hydrolyse enzymatique poussée de façon à obtenir un second hydrolysat renfermant du fer hémique complexé à des petits peptides, ainsi que des populations péptidiques non complexées au fer hémique,
- on centrifuge le second hydrolysat ainsi obtenu afin d'éliminer une proportion importante des petits peptides non liés à l'hème, et d'obtenir un culot de centrifugation très concentré en hème,
- on solubilise le culot de centrifugation notamment par addition de soude de façon à obtenir une solution extrinsèque d'hème dans laquelle l'hème est lié à des peptides de petite taille
- on mélange la solution d'hydrolysat enrichi en complexes fer hémique - peptides et la solution extrinsèque d'hème de façon à permettre la fixation de l'hème extrinsèque sur les peptides de grande taille de la solution d'hydrolysat et la libération des petits peptides liés à l'hème extrinsèque,
- on centrifuge le mélange ainsi obtenu afin d'éliminer l'hème extrinsèque qui n'a pas été fixé aux peptides de grande taille, et
- on enrichit la solution ainsi obtenue en complexes fer hémique - peptides par ultrafiltration de façon à éliminer des petits peptides libérés lors de l'étape de mélange.

2. Procédé selon la revendication 1,
**caractérisé en ce que**
l'on sèche la fraction de peptides hémiques à teneur élevée en fer de façon à obtenir une préparation alimentaire sous forme de poudre adaptée au traitement de la carence en fer.

3. Procédé selon l'une quelconque des revendications 1 et 2,
**caractérisé en ce que**
l'on effectue l'hydrolyse enzymatique ménagée et l'hydrolyse enzymatique poussée des deux fractions de sang d'animaux de départ avec une enzyme active à pH acide, notamment de la pepsine porcine.

4. Procédé selon la revendication 3,
**caractérisé en ce que**
l'on met en oeuvre l'hydrolyse enzymatique ménagée et l'hydrolyse enzymatique poussée à un pH inférieur à 6.

5. Procédé selon l'une quelconque des revendications 1 à 4,
**caractérisé en ce que**
la matière première de départ est du cruor et lors de l'étape d'hydrolyse enzymatique ménagée on ajuste le degré d'hydrolyse à une valeur inférieure ou égale à 5 %, alors que lors de l'étape d'hydrolyse enzymatique poussée, on ajuste le degré d'hydrolyse à au moins 15 %.

6. Procédé selon l'une quelconque des revendications 1 à 5,
**caractérisé en ce que**
lors de l'étape de mélange de la solution d'hydrolysat enrichie en complexes fer hémique - peptides et de la solution extrinsèque d'hème on met en oeuvre des quantités de solutions telles que la quantité d'hème apportée par la solution d'hème extrinsèque soit jusqu'à dix fois plus importante que la quantité d'hème de la solution d'hydrolysat.

7. Procédé selon l'une quelconque des revendications 1 à 6,
**caractérisé en ce que**
lors des étapes d'enrichissement par ultrafiltration, on utilise des membranes hydrophiles ayant un seuil de coupure d'environ 10 kDa.

8. Procédé selon l'une quelconque des revendications 1 à 7,
**caractérisé en ce que**
après chacune des étapes d'enrichissement par ultrafiltration, on met en oeuvre au moins une, de préférence trois étapes de dia filtration sur des membranes identiques de façon à augmenter l'enrichissement de l'hydrolysat en poursuivant l'élimination des peptides.

9. Procédé selon l'une quelconque des revendications 1 à 8,
**caractérisé en ce que**
avant l'enrichissement par ultrafiltration du premier hydrolysat, on centrifuge cet hydrolysat de façon à éliminer les peptides insolubles.

10. Préparation alimentaire à base de fer hémique adaptée au traitement de la carence en fer susceptible d'être obtenue suite à la mise en oeuvre du procédé selon l'une quelconque des revendications 1 à 9,
**caractérisée en ce qu'**
elle se présente sous la forme d'un liquide ou d'une poudre de peptides hémiques ayant un rapport pondéral fer / peptides supérieur à 2 %, de préférence d'environ 3 %, plus de 80 % de ce fer étant complexé aux peptides.

11. Préparation alimentaire à base de fer hémique adaptée au traitement de la carence en fer susceptible d'être obtenue suite à la mise en oeuvre du procédé selon l'une quelconque des revendications 1 à 9,
**caractérisée en ce qu'**
elle se présente sous la forme de gélules ayant notamment une contenance volumique d'environ 0,5 ml qui sont remplies d'une poudre de peptides hémiques selon la revendication 10.

12. Préparation alimentaire selon la revendication 11,
**caractérisée en ce que**
les gélules sont gastro protégées.

## Patentansprüche

1. Biologisches Verfahren zur Herstellung eines Hämeisenpräparats, das für die Behandlung von Eisenmangel geeignet ist, aus Tierblut, wie zum Beispiel aus Schweine- oder Rinderblut oder auch aus deren Derivaten, wie Blutkuchen,
**gekennzeichnet durch** die Abfolge der folgenden Schritte;
- eine erste Tierblutfraktion wird einer partiellen enzymatischen Hydrolyse unterzogen, um ein erstes Hydrolysat zu gewinnen, das Hämeisen enthält, das an Peptiden großer Größe komplexiert ist, sowie Peptidpopulationen, die nicht an Hämeisen komplexiert sind,
- das erste Hydrolysat, das so hergestellt wurde, wird **durch** Ultrafiltration mit Hämeisen-Peptid-Komplexen angereichert, um die nicht an dem Hämeisen komplexierten Peptidpopulationen zu entfernen, und um eine mit Hameisen-Peptid-Komplexen angereicherte Hydrolysatlösung herzustellen,
- gleichzeitig wird eine zweite Tierblutfraktion einer vollständigen enzymatischen Hydrolyse unterzogen, um ein zweites Hydrolysat herzustellen, das Hämeisen enthält, das an kleine Peptide komplexiert ist, sowie Peptidpopulationen, die nicht an Hämeisen komplexiert sind,
- das zweite Hydrolysat, das so hergestellt wurde, wird zentrifugiert, um einen großen Anteil der nicht an das Häm gebundenen kleinen Peptide zu entfernen, und um ein sehr konzentriertes Hämzentrifugat herzustellen,
- das Zentrifugat wird insbesondere **durch** die Zugabe von Natron gelöst, um eine extrinsische Hämlösung herzustellen, in der das Häm an Peptide kleiner Größe gebunden ist,
- die Hydrolysatlösung, die mit Hämeisen-Peptid-Komplexen angereicht ist, und die extrinsische Hämlösung werden gemischt, um die Fixierung des extrinsischen Häms an den Peptiden großer Größe der Hydrolysatlösung und die Freisetzung der kleinen Peptide, die an das extrinsische Häm gebunden sind, zu ermöglichen,
- die Mischung, die so hergestellt wurde, wird zentrifugiert, um das extrinsische Häm zu entfernen, das sich nicht an den Peptiden mit großer Größe fixiert hat, und
- die Lösung, die so hergestellt wurde, wird **durch** Ultrafiltration mit Hämeisen-Peptid-Komplexen angereichert, um die kleinen Peptide zu entfernen, die beim Schritt des Mischens freigesetzt werden.

2. Verfahren nach Anspruch 1,
**dadurch gekennzeichnet, dass**
die Fraktion der Hämpeptide, die einen hohen Eisengehalt haben, getrocknet wird, um ein Nahrungsmittelpräparat in Form von Pulver, das für die Behandlung von Eisenmangel geeignet ist, herzustellen.

3. Verfahren nach irgendeinem der Ansprüche 1 und 2,
**dadurch gekennzeichnet, dass**
eine partielle enzymatische Hydrolyse und eine vollständige enzymatische Hydrolyse der beiden Ausgangstierblutfraktionen mit einem aktiven Enzym mit sauren pH-Wert, insbesondere mit einem Schweinepepsin, durchgeführt wird.

4. Verfahren nach Anspruch 3,
**dadurch gekennzeichnet, dass**
die partielle enzymatische Hydrolyse und die vollständige enzymatische Hydrolyse mit einem pH-Wert von weniger als 6 eingesetzt wird.

5. Verfahren nach irgendeinem der Ansprüche 1 bis 4,
**dadurch gekennzeichnet, dass**
der Ausgangsrohstoff Blutkuchen ist und bei dem Schritt der partiellen enzymatischen Hydrolyse der Hydrolysegrad auf einen Wert kleiner oder gleich 5 % eingestellt wird, während beim Schritt der vollständigen enzymatischen Hydrolyse, der Hydrolysegrad auf weniger als 15 % eingestellt wird.

6. Verfahren nach irgendeinem der Ansprüche 1 bis 5,
**dadurch gekennzeichnet, dass**
bei dem Schritt der Mischung der Hydrolysatlösung, die mit Hämeisen-Peptid-Komplexen angereichert ist, und der extrinsischen Hämlösung, die Mengen der Lösung so eingesetzt werden, dass die Hämmenge die von der extrinsischen Hämlösung eingebracht wird, bis zu zehn Mal größer ist als die Hämmenge der Hydrolysatlösung.

7. Verfahren nach irgendeinem der Ansprüche 1 bis 6,
**dadurch gekennzeichnet, dass**
bei den Schritten der Anreicherung durch Ultrafiltration hydrophile Membranen mit einer Abscheidegrenze von etwa 10 kDa verwendet werden.

8. Verfahren nach irgendeinem der Ansprüche 1 bis 7,
**dadurch gekennzeichnet, dass**
nach jedem der Anreicherungsschritte durch Ultrafiltration, mindestens ein, vorzugsweise drei Schritte der Diafiltration durch identische Membranen eingesetzt werden, um die Anreicherung des Hydrolysats zu erhöhen, wobei danach gestrebt wird, die Peptide zu entfernen.

9. Verfahren nach irgendeinem der Ansprüche 1 bis 8,
**dadurch gekennzeichnet, dass**
vor der Anreicherung durch Ultrafiltration des ersten Hydrolysats, dieses Hydrolysat zentrifugiert wird, um die unlöslichen Peptide zu entfernen.

10. Hämeisenpräparat, das zur Behandlung von Eisenmangel geeignet ist, das nach dem Einsatz eines Verfahrens nach irgendeinem der Ansprüche 1 bis 9 hergestellt werden kann,
**dadurch gekennzeichnet, dass**
es sich in Form einer Flüssigkeit oder eines Pulvers aus Hämpeptiden mit einem Gewichtsverhältnis Eisen/Peptide größer als 2 %, vorzugsweise etwa 3 %, darstellt, wobei mehr als 80 % dieses Eisens an den Peptiden komplexiert ist.

11. Hämeisenpräparat, das zur Behandlung von Eisenmangel geeignet ist, das nach dem Einsatz eines Verfahrens nach irgendeinem der Ansprüche 1 bis 9 hergestellt werden kann, **dadurch gekennzeichnet, dass**
es sich in Form von Gelatinekapseln darstellt, welche insbesondere eine Volumenkapazität von etwa 0,5 ml haben, die mit einem Hämpeptidpulver nach Anspruch 10 gefüllt sind.

12. Nahrungsmittelpräparat nach Anspruch 11,
**dadurch gekennzeichnet, dass**
die Gelatinekapseln magensäureresistent sind.

## Claims

1. Biological method of obtaining a food preparation based on heme iron, suitable for the treatment of iron deficiency, from animals' blood, e.g. pig's blood or bull's blood, or its derivatives such as cruor, **characterized by** the following series of steps:
- a first fraction of animals' blood is subjected to a controlled enzymatic hydrolysis to give a first hydrolysate containing heme iron complexed with large peptides, and peptide populations not complexed with the heme iron,
- the resulting first hydrolysate is enriched in heme iron/peptide complexes by ultrafiltration, which removes peptide populations not complexed with the heme iron to give a hydrolysate solution enriched in heme iron/peptide complexes,
- a second fraction of animals' blood is subjected in parallel to a thorough enzymatic hydrolysis to give a second hydrolysate containing heme iron complexed with small peptides, and peptide populations not complexed with the heme iron,
- the resulting second hydrolysate is centrifuged to remove a substantial proportion of the small peptides not bound to the heme to give a centrifugation residue highly concentrated in heme,
- the centrifugation residue is solubilized, especially by adding sodium hydroxide solution, to give an extrinsic heme solution in which the heme is bound to small peptides,
- the hydrolysate solution enriched in heme iron/peptide complexes and the extrinsic heme solution are mixed so as to allow fixing of the extrinsic heme to the large peptides of the hydrolysate solution and freeing of the small peptides bound to the extrinsic heme,
- the resulting mixture is centrifuged to remove the extrinsic heme that was not fixed to the large peptides, and
- the resulting solution is enriched in heme iron/peptide complexes by ultrafiltration, which removes small peptides freed in the mixing step.

2. Method according to Claim 1, **characterized in that** the fraction of heme peptides with a high iron content is dried to give a food preparation in powder form that is suitable for the treatment of iron deficiency.

3. Method according to Claim 1 or 2, **characterized in that** the controlled enzymatic hydrolysis and the thorough enzymatic hydrolysis of the two initial fractions of animals' blood are performed with an enzyme that is active at acidic pH, especially porcine pepsin.

4. Method according to Claim 3, **characterized in that** the controlled enzymatic hydrolysis and the thorough enzymatic hydrolysis are performed at a pH below 6.

5. Method according to any one of Claims 1 to 4, **characterized in that** the starting material is cruor and in the controlled enzymatic hydrolysis step the degree of hydrolysis is adjusted to a value below or equal to 5%, whereas in the thorough enzymatic hydrolysis step the degree of hydrolysis is adjusted to at least 15%.

6. Method according to any one of Claims 1 to 5, **characterized in that** the amounts of solutions used in the step in which the hydrolysate solution enriched in heme iron/peptide complexes is mixed with the extrinsic heme solution are such that the amount of heme introduced by the extrinsic heme solution is up to ten times greater than the amount of heme in the hydrolysate solution.

7. Method according to any one of Claims 1 to 6, **characterized in that** hydrophilic membranes with a cut-off limit of about 10 kDa are used in the ultrafiltration enrichment steps.

8. Method according to any one of Claims 1 to 7, **characterized in that**, after each of the ultrafiltration enrichment steps, at least one and preferably three diafiltration steps are carried out on identical membranes to increase the enrichment of the hydrolysate by continuing to remove the peptides.

9. Method according to any one of Claims 1 to 8, **characterized in that**, before enrichment of the first hydrolysate by ultrafiltration, said hydrolysate is centrifuged to remove the insoluble peptides.

10. Food preparation based on heme iron, suitable for the treatment of iron deficiency, obtainable after carrying out the method according to any one of Claims 1 to 9, **characterized in that** it takes the form of a liquid or a powder of heme peptides with a weight ratio iron/peptides of more than 2%, preferably of about 3%, more than 80% of this iron being complexed with the peptides.

11. Food preparation based on heme iron, suitable for the treatment of iron deficiency, obtainable after carrying out the method according to any one of Claims 1 to 9, **characterized in that** it takes the form of capsules with a volume capacity of about 0.5 ml in particular, which are filled with a powder of heme peptides according to Claim 10.

12. Food preparation according to Claim 11, **characterized in that** the capsules are gastroprotected.
